# EUROPEAN PATENT APPLICATION

(11) **EP 0 768 530 A1**
(43) Date of publication of application: **16.04.1997**
(21) Application number: 95116306.2
(22) Date of filing: 16.10.1995
(51) Int. Cl.: G01N 33/546, G01N 33/58

(54) **Process for assaying biological substance**

(71) Applicant: NIPPON PAINT CO., LTD., Osaka-shi Osaka 530 (JP)
(72) Inventor: Kobayashi, Hisaka, London NW3 3EJ (GB); Takahashi, Yoji, Tokyo (JP)
(74) Representative: Hansen, Bernd, Dr. Dipl.-Chem.

(57) **Abstract**

A process for assaying a biological substance is provided. In the assay, a reagent comprising magnetic particles having loaded thereon a substance that binds to the target substance is utilized; non-magnetic colored particles; and magnetic particles having loaded thereon a substance that binds to an assay interfering substance is utilized. The assay is carried out by mixing the reagent with the body fluid; magnetically removing the magnetic particles and the substances that were bound to the magnetic particles; and measuring the amount of the remaining non-magnetic colored particles. Even when the body fluid specimen is measured with no pretreatment, measurements of high sensitivity and high reliability can be obtained in a short period without being interfered with the assay interfering substances in the body fluid.

## Description

### FIELD OF THE INVENTION

This invention is an improvement in the process for assaying a biological substance by means of an immunoreaction utilizing magnetic particles and non-magnetic particles. More particularly, this invention relates to a process for assaying a target biological substance that enables use of a body fluid for the specimen, and that is capable of obtaining measurements of high sensitivity and high reliability in a quite short period without being influenced by the substances in the body fluid that interferes with the assay.

### BACKGROUND OF THE INVENTION

Use of magnetic particles in an immunoassay comprising the step of reacting an antigen with an antibody is disclosed in USP 4177253.

A method for assaying an antigen or an antibody comprising the steps of loading an antibody or an antigen on insoluble carrier particles, and allowing the thus loaded antibody or antigen to react with an antigen or an antibody or a mixture thereof in a liquid medium to measure the increase/decrease of the reaction product is disclosed, for example, in Japanese Patent Application Laid-Open No. 1-193647. In this method, the reaction is effected by using magnetic and non-magnetic particles for the carrier particles; a magnetic field is applied to remove the magnetic particles including the magnetic particles that failed to react from the reaction mixture; and the remaining non-magnetic particles are detected to thereby qualitatively or quantitatively determine the antigen or the antibody in the liquid medium.

In the methods as described above, a reliable, accurate measurement could be effected only when the specimen used for the assay is a biological substance from which the substances that interfere with the assay had been removed.

As a matter of fact, assay at a high sensitivity could be carried out only after separating the serum since erythrocytes, leukocytes, platelets, hemoglobin, bilirubin, lipoproteins and the like in the whole blood interfered with the assay. Such situation is undesirable since there is a need for a highly reliable accelerated assay of a biological substance at a high sensitivity that requires no pretreatment such as serum separation of the specimen used for the assay of the biological substance. A pretreatment such as serum separation for the purpose of removing the assay interfering substance should make an accelerated assay difficult to be carried out.

In view of such situation, an object of the present invention is to obviate the above-described problems of the prior art and develop a highly reliable process for qualitatively or quantitatively determining a biological substance that exhibits a high sensitivity.

### SUMMARY OF THE INVENTION

According to the present invention, there is provided a process for assaying a biological substance characterized in that said process comprises the steps (1) to (4) carried out in this order:
(1) the step of mixing a body fluid with particles (a), (b) and (c):
   (a) magnetic particles having loaded thereon a substance that binds to an assay interfering substance in the body fluid through an immunoreaction;
   (b) magnetic particles having loaded thereon a substance that binds to the target substance in the body fluid through an immunoreaction; and
   (c) non-magnetic colored particles having loaded thereon a substance that binds to the target substances in the body fluid through an immunoreaction;
(2) the step of applying magnetic field to the mixture;
(3) the step of removing the complexes formed by the immunoreaction of the magnetic particles and the magnetic particles that failed to react by means of magnetism; and
(4) the step of determining the amount of the remaining colored particles by measuring their absorption at a wave length of 350 nm or longer to thereby qualify or quantify the target substance in the body fluid without being influenced by the interfering substances in the body fluid.

In the process of the invention, the magnetic particles may preferably comprise at least one polymer selected from polystyrenes or (meth)acrylates, and have a coating layer of an iron oxide ferrite on its surface. The magnetic particles may have a diameter of from 0.1 to 5 µm, and preferably, 0.2 to 3 µm.

In addition, it is preferable that the body fluid is whole blood and the assay interfering substance is erythrocyte; the colored particles are colored particles of one or more types having a diameter of from 0.1 to 5 µm and having at least one color selected from white, black, red, blue, yellow and a color formed by mixing such colors; and the wave length used for measuring the absorbance is in the range of from 350 to 1000 nm.

### BRIEF DESCRIPTION OF THE DRAWINGS

[FIG. 1] This figure is a schematic view for explaining the process of the invention.

[FIG. 2] This figure is a schematic view showing an example of the calibration curve used for the present invention.

### DETAILED DESCRIPTION OF THE INVENTION

The target substance to be assayed by the process of the present invention is a biological substance found in human or an animal, and the specimen to be evaluated is a body fluid such as blood or urine containing such a substance.

A body fluid may contain biological substances such as erythrocytes, hemoglobin, bilirubin, and the like as well as drugs such as phenobarbital, phenytoin, digoxin, imipramine, theophylline, penicillin, and the like, and such a substance in the specimen to be determined should interfere with the assay to adversely affect the sensitivity and the accuracy of the assay.

The biological substance to be assayed in the present invention is a biological substance that is a particular antigen or antibody present in the body fluid to be assayed. Exemplary such biological substances are as described below.

Antigens: IgG, IgA, IgM, IgE, albumin, HCG, AFP, cardiolipin antigen, blood group substances, concanavalin A, DNT, prostaglandin, CRP, HBs, human growth hormone, steroid hormone, CEA, IgD, Lp(a), Apo-AI, Apo-AII, Apo-CII, Apo-CIII, Apo-B, Apo-E, hemoglobin, etc.

Antibodies: anti-albumin antibodies such as anti-albumin antibody, anti-AFP antibody, etc.; anti-globulin antibodies such as anti-HCG antibody, anti-IgG antibody, anti-IgA antibody, anti-IgM antibody, anti-IgE antibody, anti-IgD antibody, anti-CRP antibody, etc.; anti-hormone antibodies such as anti-human growth hormone antibody, anti-steroid antibody, etc.; anti-DNT antibody, anti-prostaglandin antibody, anti-human coagulation factor antibody, anti-HBs antibody, etc.

In the process of the present invention, particles (a), (b) and (c) as described below are employed.
(a) magnetic particles having loaded thereon a substance that binds to an assay interfering substance in the body fluid through an immunoreaction;
(b) magnetic particles having loaded thereon a substance that binds to the target substance in the body fluid through an immunoreaction; and
(c) non-magnetic colored particles having loaded thereon a substance that binds to the target substances in the body fluid through an immunoreaction.

The non-magnetic particles that can be used in the present invention may comprise an organic high-molecular weight substance or an inorganic substance. Exemplary organic high-molecular weight substances include gelatin particles; and latices of organic high-molecular weight substances such as polystyrene and styrene-butadiene copolymer prepared by emulsion polymerization, and use of polystyrene latex and polyvinyltoluene latex is advantageous. Use of at least one polymer of polystyrene and (meth)acrylate is also possible.

Exemplary (meth)acrylates that can be used include 2-hydroxyethyl (meth)acrylate, 2-hydroxypropyl (meth)acrylate, 1-methyl-2-hydroxyethyl (meth)acrylate, glycerol monomethacrylate, 2-acrylamide-2-methylpropanesulfonate, 2-sulfoethyl methacrylate, acid phosphoxyethyl methacrylate, 3-chloro-2-acid phosphoxyethyl methacrylate, acid phosphoxypropyl methacrylate, ethyl (meth)acrylate, n-butyl (meth)acrylate, i-butyl (meth)acrylate, 2-ethylhexyl (meth)acrylate, lauryl methacrylate, cyclohexyl methacrylate, (meth)acrylamide, N-methylol acrylamide, N-butoxymethylacrylamide, glycidyl (meth)acrylate, methylglycidyl (meth)acrylate, etc.

Particle size is not limited to any particular range, and may preferably be in the range of from 0.01 to 100 µm, and more preferably, from 0.1 to 30 µm. The particles of such particle size are preferable for immunoreaction since such particles should have sufficient floatability in an aqueous solution, and would not precipitate within short period.

The non-magnetic particles are colored by addition of a colorant. The colorant used may preferably be the one that would enable measurement of the absorption at a wave length of from 350 to 1,000 nm.

The magnetic particles used in the present invention are those prepared by incorporating iron or an iron oxide in the above-described particles of an organic high-molecular weight substance or an inorganic substance, or by coating the nucleus of the above-described particles of an organic high-molecular weight substance or an inorganic substance with a ferrite to thereby produce ferrite-coated particles.

In the present invention, the magnetic particles may be those prepared by further treating the magnetic particles that have been prepared as described above with a high-molecular compound. The high-molecular compounds that may be used for such treatment include silanes, nylon and polystyrene.

The particles as described above are loaded with a substance that immunologically binds to the target substance to be assayed or the assay interfering substance in the body fluid. When the target substance or the assay interfering substance is a particular antigen, the substance that immunologically binds to such substance is an antibody, and when the target substance or the assay interfering substance is a particular antibody, the substance that immunologically binds to such substance is an antigen.

The procedure for loading the particles with the immunologically binding substance does not differ whether the particles are magnetic or non-magnetic, and an antibody or an antigen is physically adsorbed on the particles or chemically loaded on the particles. Physical adsorption may be carried out by reacting said particles with an antigen or an antibody in an adequate buffer solution. The buffer solutions that may be used in such reaction include phosphate buffer saline, Tris-HCl buffer solution, carbonate buffer solution. The reaction will readily proceed when the particles are mixed with the antigen or the antibody at room temperature, and the desired antigen or antibody-loaded particles will be produced. Chemical loading may be carried out by employing carbodiimide method or glutaraldehyde method used in the so-called peptide binding process.

The antibodies loaded on the particles may be either polyclonal or monoclonal, and may be in the form of γ-globulin, IgG, IgM, F(ab')2, Fab', or the like.

When the substance to be loaded is an antigen, it may be a cell debris, a hapten, an antigenic protein, an immunocomplex, a natural or synthetic high-molecular weight antigen.

The amount of the antigen or the antibody loaded on the particles may considerably differ by such factor as the type of the colored particles, and in general, an adequate amount is selected in the range of from 0.001 mg/ml to 20 mg/ml, and preferably, from 0.005 mg/ml to 5 mg/ml.

The particles that have been loaded with a substance that undergoes the immunoreaction are then dispersed in an aqueous medium such as a buffer solution to 0.01 to 10% by weight, and the particles are used as a suspension of latex particles.

In the assay of the present invention, the particles (a), (b) and (c) as described above are mixed with the body fluid in its step (1).

To 1 volume of non-magnetic colored particles (c) having loaded thereon a substance that immunologically binds to the target substances is used 1/4 to 4 volumes of magnetic particles (b) having loaded thereon a substance that immunologically binds to the target substance; and 1/10 to 10 volumes of magnetic particles (a) having loaded thereon a substance that immunologically binds to an assay interfering substance in the body fluid through an immunoreaction.

The particles (a), (b) and (c) and the body fluid may be mixed in an arbitrary order, and it is possible to mix the magnetic particles (a) and (b) with the non-magnetic particles (c), and then mix the particle mixture with the body fluid; or to mix the non-magnetic particles (c) with the body fluid, and further with the magnetic particles (a) and (b). It is preferable to mix the magnetic particles (a) and (b) with the non-magnetic particles (c), and then mix the particle mixture with the body fluid.

If necessary, the specimen may be diluted with a buffer or the like.

The mixing procedure is generally carried out at room temperature. However, reaction system may be heated to 30 to 40°C to promote the immunoreaction, or alternatively, cooled to 4 to 20°C for maintaining stability of the reagents.

The step (2) of the assay of the present invention is the step of applying magnetic field to the mixture obtained in step (1).

Intensity of the magnetic field is not limited to any particular range, and typical intensity is in the range of 1,000 to 10,000 Gauss when the total volume of the mixture is 200 µl. A commercially available magnetic gatherer such as GATHERIN manufactured by Nippon Paint Co., Ltd. may be employed.

In the next step (3), the magnetic particles (a) and (b) that failed to react, and the complexes formed by the immunoreaction of the magnetic particles (a) and (b) with particle (c) or with other substance are removed by means of magnetism. The step (3) may be carried out for a non-limited period of time, and typically for 10 seconds to 5 minutes.

In step (4), amount of the colored particles (c) which was not magnetically removed is determined by measuring absorption at a wave length of 350 nm or longer, and preferably, from 500 to 700 nm. It is preferable to effect the measurement by using a wave length of the range that is absorbed by the colorant incorporated in the colored particles. The amount of the remaining colored particles (c) that survived the magnetic removal negatively depends on the content of the target substance in the specimen.

It would be generally preferable to depict a calibration curve by measuring specimens containing different known amounts of the target substance for their absorbance, and concentration of the target substance in the specimen of unknown concentration is determined by referring the calibration curve after measuring the absorbance of the specimen of unknown concentration.

An embodiment of determining the amount of CRP (C-reactive protein) in whole blood is described by referring to the drawing. It should be noted that CRP is a β-globulin in serum whose amount increases in such occasion as inflammatory diseases accompanying tissue damage.

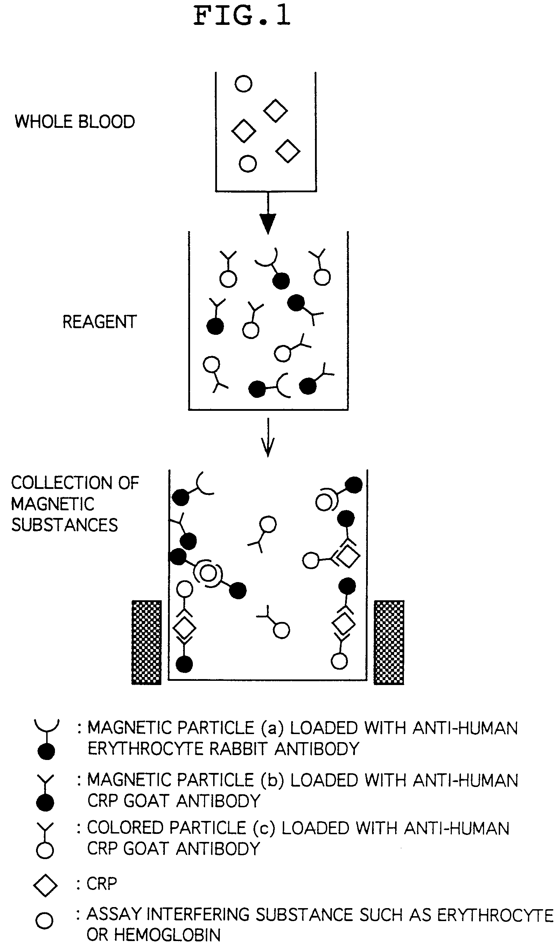

FIG. 1 shows the case wherein the body fluid assayed is whole blood. As shown in FIG. 1, whole blood contains CRP which is the substance to be assayed together with erythrocytes, hemoglobin, and the like that function as the assay interfering substances. The reagent used for the assay is a mixture of (a) magnetic particles having loaded thereon an anti-human erythrocyte rabbit antibody ((a) magnetic particles having loaded thereon a substance that binds to an assay interfering substance in the body fluid through an immunoreaction); (b) magnetic particles having loaded thereon an anti-human CRP goat antibody ((b) magnetic particles having loaded thereon a substance that binds to the target substance in the body fluid through an immunoreaction); and (c) colored particles having loaded thereon an anti-human CRP goat antibody ((c) non-magnetic colored particles having loaded thereon a substance that binds to the target substances in the body fluid through an immunoreaction.

When the whole blood is mixed with the reagent, and magnetic field is applied to the mixture, the magnetic particles are collected as shown in the drawing.

The particles collected are particle (a); immunocomplex of particle (a) with the assay interfering substance; particle (b); the CRP bound to particle (b); and particle (c) bound to the CRP bound to particle (b). The residual particles that are not magnetically collected are the colored particles (c) having loaded thereon an antihuman CRP goat antibody, which failed to bind to the CRP.

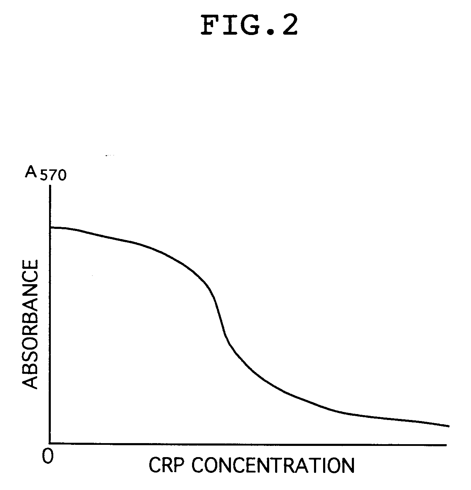

Shown in FIG. 2 is a schematic calibration curve depicted for blood of known CRP concentrations by measuring the absorbance at a wave length of 570 nm. Since the specimen like blood can be assayed with no pretreatment such as adjustment of the blood components by such means as serum separation, an accelerated assay advantageous for such occasions as bedside assay and emergency assay is realized. Capability of the use of the as-collected whole blood for the specimen is particularly advantageous for case of the bedside assay.

The assay of the present invention is an immunoassay utilizing magnetic particles. The assay of the present invention, however, may be modified as an EIA, luminescence assay, or the like.

### EXAMPLES

The present invention is hereinafter described by referring to the Examples, which by no means limit the scope of the invention.

### (Example 1)

### [Preparation of reagent]

### Preparation of anti-CRP immobilized colored particles (c)

Colored particles of 0.2 µm (colored particles of a styrene polymer prepared by suspension polymerization) was dispersed in 1 ml of 20mM phosphate buffer saline, pH 7.4 to a solid content of 1%.

To the dispersion was then added 0.25 mg of anti-human CRP goat antibody, and the dispersion was stirred. The particles were immobilized by storing the dispersion in a refrigerator for 5 days. The dispersion was subjected to centrifugation (12,000 rpm x 20 min) to remove the supernatant, and the thus separated colored particles were resuspended in 20mM phosphate buffer saline, pH 7.4 containing 1 ml 1% bovine serum albumin. The procedure was repeated three times.

### Preparation of anti-CRP immobilized magnetic particles (b)

Magnetic particles of 2 µm (prepared by styrene polymer particles prepared by suspension polymerization with ferrite coating) was dispersed in 1 ml of 20mM phosphate buffer saline, pH 7.4 to a solid content of 1%. To the dispersion was then added 0.25 mg of anti-human CRP goat antibody, and the dispersion was stirred. The particles were immobilized by storing the dispersion in a refrigerator for 3 days. The dispersion was treated in a magnetic separator to remove the supernatant, and the thus separated magnetic particles were resuspended in 20mM phosphate buffer saline, pH 7.4 containing 1 ml 1% bovine serum albumin. The procedure was repeated three times.

### Preparation of anti-human erythrocyte immobilized magnetic particles (a)

Magnetic particles of 2 µm (which are similar to the above-described particles) was dispersed in 1 ml of 20mM phosphate buffer saline, pH 7.4 to a solid content of 1%. To the dispersion was then added 0.25 mg of anti-human erythrocyte rabbit antibody, and the dispersion was stirred. The particles were immobilized by storing the dispersion in a refrigerator for 3 days. The dispersion was treated in a magnetic separator to remove the supernatant, and the thus separated magnetic particles were resuspended in 20mM phosphate buffer saline, pH 7.4 containing 1 ml 1% bovine serum albumin. The procedure was repeated three times.

### [Selection of the wave length employed for measurement]

The wave length absorbed by the colored particles was detected by scanning the wave length. The wave length used for the measurement was the one absorbed by the colorant of the colored particles.

### [Procedure]

1. Colored particles (c) immobilized with anti-CRP, magnetic particles (b) immobilized with anti-CRP, and magnetic particles (a) immobilized with anti-human erythrocyte were mixed at a ratio of 1:2:1, and the mixture was used for the particle preparation.
2. Standard CRP (25.3 mg/dl) was diluted to different concentrations, and the dilutions were respectively mixed with blood. The mixture was used for the specimen.
3. 1 µl of the specimen and 200 µl of the reagent prepared from the particles were dispensed in the wells of 96 well microplate, and the mixture was stirred, and allowed to react for 5 minutes at room temperature.
4. A magnetic particle-gathering apparatus (GATHERIN, manufactured by Nippon Paint Co., Ltd.) was combined with the microplate, and the immunocomplex of the magnetic particle (b) and the colored particle (c) bound to each other with the intervening CRP antigen; the immunocomplex of the magnetic particles (a) bound to each other with the intervening erythrocyte; and the magnetic particles (a) and (b) that failed to react were magnetically collected.
5. The concentration of the remaining colored particles (c) was determined with a microplate reader by measuring absorbance at a wave length of 570 nm and a calibration curve was depicted.

### (Comparative Example)

### Preparation of anti-CRP immobilized colored particles (c)

Colored particles of 0.2 µm was dispersed in 1 ml of 20mM phosphate buffer saline, pH 7.4 to a solid content of 1%.

To the dispersion was then added 0.25 mg of anti-human CRP goat antibody, and the dispersion was stirred. The particles were immobilized by storing the dispersion in a refrigerator for 3 days. The dispersion was subjected to centrifugation (12,000 rpm x 20 min.) to remove the supernatant, and the thus separated colored particles were resuspended in 20mM phosphate buffer saline, pH 7.4 containing 1 ml 1% bovine serum albumin. The procedure was repeated three times.

### Preparation of anti-CRP immobilized magnetic particles (b)

Magnetic particles of 2 µm was dispersed in 1 ml of 20mM phosphate buffer saline, pH 7.4 to a solid content of 1%. To the dispersion was then added 0.25 mg of anti-human CRP goat antibody, and the dispersion was stirred. The particles were immobilized by storing the dispersion in a refrigerator for 3 days. The dispersion was treated in a magnetic separator to remove the supernatant, and the thus separated magnetic particles were resuspended in 20mM phosphate buffer saline, pH 7.4 containing 1 ml 1% bovine serum albumin. The procedure was repeated three times.

### [Selection of the wave length employed for measurement]

The wave length absorbed by the colored particles was detected by scanning the wave length. The wave length used for the measurement was the one absorbed by the colorant of the colored particles.

### [Procedure]

1. Colored particles (c) immobilized with anti-CRP and magnetic particles (b) immobilized with anti-CRP were mixed at a ratio of 1:2, and the mixture was used for the particle preparation.
2. Standard CRP (25.3 mg/dl) was diluted to different concentrations, and the dilutions were respectively mixed with blood. The mixture was used for the specimen.
3. 1 µl of the specimen and 200 µl of the reagent prepared from the particles were dispensed in the wells of 96 well microplate, and the mixture was stirred, and allowed to react for 5 minutes at room temperature.
4. A magnetic particle-gathering apparatus (GATHERIN, manufactured by Nippon Paint Co., Ltd.) was combined with the microplate, and the immunocomplex of the magnetic particle (b) and the colored particle (c) bound to each other with the intervening CRP antigen; and the magnetic particles (b) that failed to react were magnetically collected.
5. The concentration of the remaining colored particles (c) was determined with a microplate reader by measuring absorbance at a wave length of 570 nm and a calibration curve was depicted.

In Example 1, magnetic particles (a) that had been immobilized with the anti-human erythrocyte antibody were present in the reagent prepared from particles, and therefore, when the blood was used for the specimen, the absorbance decreased with the increase in the antigen concentration to enable assay of the antigen concentration at a high accuracy. On the other hand, no decrease in the absorbance with the increase in the antigen concentration was observed in Comparative Example.

The process of the present invention is an improvement in the immunoassay employing the magnetic and non-magnetic particles. Since the process of the present invention involves the addition of the magnetic particles having loaded thereon a substance that binds to the assay interfering substance in the body fluid to be assayed, an accelerated assay at a high sensitivity with no influence of the assay interfering substance is enabled even when the sample is an untreated body fluid.

## Claims

1. A process for assaying a biological substance characterized in that said process comprises the steps (1) to (4) carried out in this order:
(1) the step of mixing a body fluid with particles (a), (b) and (c):
(a) magnetic particles having loaded thereon a substance that binds to an assay interfering substance in the body fluid through an immunoreaction;
(b) magnetic particles having loaded thereon a substance that binds to the target substance in the body fluid through an immunoreaction; and
(c) non-magnetic colored particles having loaded thereon a substance that binds to the target substances in the body fluid through an immunoreaction;
(2) the step of applying magnetic field to the mixture;
(3) the step of removing the complexes formed by the immunoreaction of the magnetic particles and the magnetic particles that failed to react by means of magnetism; and
(4) the step of determining the amount of the remaining colored particles by measuring their absorption at a wave length of 350 nm or longer to thereby qualify or quantify the target substance in the body fluid without being influenced by the interfering substances in the body fluid.

2. A process for assaying a biological substance according to claim 1 wherein said magnetic particles are particles comprising at least one polymer selected from polystyrenes and (meth)acrylates having a coating layer of an iron oxide ferrite on its surface, and said particles have a diameter of from 0.2 to 3 µm.

3. A process for assaying a biological substance according to claim 1 or 2 wherein said body fluid is whole blood, and said an assay interfering substances is erythrocyte, leukocyte, platelet, hemoglobin, bilirubin, or a lipoprotein.

4. A process for assaying a biological substance according to any one of claims 1 to 3 wherein said colored particles are colored particles having a diameter of from 0.1 to 5 µm and having at least one color selected from white, black, red, blue, yellow and colors formed by mixing such colors; and the wave length used for measuring the absorbance is in the range of from 350 to 1000 nm.

5. A kit for assaying a biological substance said kit comprising particles (a), (b) and (c):
(a) magnetic particles having loaded thereon a substance that binds to an assay interfering substance in the body fluid through an immunoreaction;
(b) magnetic particles having loaded thereon a substance that binds to the target substance in the body fluid through an immunoreaction; and
(c) non-magnetic colored particles having loaded thereon a substance that binds to the target substances in the body fluid through an immunoreaction.
